# EUROPEAN PATENT SPECIFICATION

(11) **EP 1 054 680 B1**
(45) Date of publication and mention of the grant of the patent: **09.03.2005**
(21) Application number: 98962163.6
(22) Date of filing: 22.12.1998
(51) Int. Cl.: A61K 35/74, A61K 31/70

(54) **TREATMENT OF BLADDER CANCER BY MYCOBACTERIUM PHLEI CELL WALL**
BEHANDLUNG DES HARNBLASENKREBSES DURCH MYCOBACTERIUM PHLEI ZELLWAND
TRAITEMENT DU CANCER DE LA VESSIE PAR LES PAROIS DE MYCOBACTERIUM PHLEI

(30) Priority: 18.02.1998 US 75111 P
(43) Date of publication of application: 29.11.2000
(73) Proprietor: Bioniche Life Sciences Inc., London, Ontario N6M 1A3 (CA)
(72) Inventor: PHILLIPS, Nigel, C., London, Ontario N6M 1A3 (CA); FILION, Mario, C., London, Ontario N6M 1A3 (CA)
(74) Representative: Viering, Jentschura & Partner
(86) International application number: PCT/CA1998/001190
(87) International publication number: WO 1999/042113

(56) References cited:
- WO-A-99/07383
- FILION, M. C. (1) ET AL: "Mycobacterial cell wall-DNA complex induces apoptosis in cancer cells." JOURNAL OF PHARMACY AND PHARMACOLOGY, (SEPT., 1998) VOL. 50, NO. SUPPL., PP. 39. MEETING INFO.: 135TH MEETING OF THE BRITISH PHARMACEUTICAL CONFERENCE EASTBOURNE, ENGLAND, UK SEPTEMBER 8-11, 1998 ISSN: 0022-3573., XP002100761
- FILION M C ET AL: "Mycobacterium phlei cell wall complex directly induces apoptosi in human bladder cancer cells." BRITISH JOURNAL OF CANCER, (1999 JAN) 79 (2) 229-35. JOURNAL CODE: AV4. ISSN: 0007-0920., XP002100762 SCOTLAND: United Kingdom
- CHIN J L ET AL: "Mycobacterium cell wall: an alternative to intravesical bacillus Calmette Guerin (BCG) therapy in orthotopic murine bladder cancer--." JOURNAL OF UROLOGY, (1996 SEP) 156 (3) 1189-93. JOURNAL CODE: KC7. ISSN: 0022-5347., XP002100763 United States
- MORALES A ET AL: "Immunotherapy of an experimental adenocarcinoma of the prostate." JOURNAL OF UROLOGY, (1995 MAY) 153 (5) 1706-10. JOURNAL CODE: KC7. ISSN: 0022-5347., XP002100764 United States

## Description

### FIELD OF INVENTION

This application claims priority of U.S. Provisional Application 60/075,111, filed February 18, 1998.

The present invention refers to the use of deproteinized delipidated Mycobacterium phlei cell wall (MCC) for the manufacture of a medicament for treatment of urinary bladder cancer in an animal including human having urinary bladder cancer. The present invention comprises the use for the manufacture of a medicament of a *Mycobacterium phlei* (*M. phlei*)-DNA (M-DNA)-*M. phlei* cell wall complex (MCC), wherein the M-DNA is preserved and complexed on the *M. phlei* cell wall such that the MCC is effective in inhibiting proliferation of and inducing apoptosis in bladder cancer cells.

### BACKGROUND OF THE INVENTION

Cancer is an aberrant net accumulation of atypical cells, which can result from an excess of proliferation, an insufficiency of apoptosis, or a combination of the two. Apoptosis can be initiated by ligands which bind to receptors on the cell surface (Muzio et al. Cell 85:817-827, 1996). Mutations in these receptors can cause a failure of apoptosis. Apoptosis also can be induced by intracellular proteins including, but not limited to, p53/p21 regulators (Levine A. Cell 88:323-331, 1997). Loss of functional p53/p21 correlates with aggressiveness in a variety of cancers (Fisher D. Cell 78:529-542, 1994). Chemotherapeutic agents rely primarily on induction of apoptosis in cancer cells for their therapeutic effect. Drug resistance, which diminishes the effectiveness of chemotherapeutic agents, leads directly or indirectly to reduced apoptosis and is generally associated with poor prognosis in a variety of cancers.

Cancer of the bladder is particularly difficult to treat successfully (Lamm et al. Journal of Urology 153:1444-1450, 1995). Preparations of bacterial origin used to treat bladder cancer include, but are not limited to, *bacillus Calmette-Guerin* (BCG) (Pryor et al. British Journal of Cancer 71:801-807, 1995) and Regressin® (Bioniche, Inc. London, Ontario, Canada), a mycobacterial cell wall extract (MCWE) formulated as a mineral oil emulsion (U.S. Patent No. 4,744;984; Chin et al. Journal of Urology 156:1189-1193. 1996).

BCG does not induce apoptosis directly (Sasaki et al. Urology International 59;142-148, 1997). Rather, BCG stimulates cells of the immune system to produce bioactive molecules such as, but not limited to, cytokines and reactive oxygen species (Kudoh et al. British Journal of Urology 8052:40, 1997), which then induce apoptosis and cytolysis.

MCWE, which is composed primarily of peptidoglycan and glycolipid containing N-acetylmuramyl-L-alanyl-D-isoglutamine and mycolic acid derivatives (Chin et al Journal of Urology 156:1189-1193, 1996), is thought to stimulate the immune system by activation of macrophage and monocyte mediated reactions (Teware et al. Veterinary Parasitology 62:223-230, 1996).

The therapeutic benefits obtained using BCG and MCWE are variable and inconsistent, and appear to depend on the method of preparation and the method of delivery and on the variability in immunogenicity and stability of the resultant preparation. Moreover, live BCG can cause serious side effects including, but not limited to, fever, setum sickness-like syndromes, granulomatous infection, sepsis and even death (Lamm et al. Journal of Urology 147:596-600, 1992),

Other prior an anti-bladder cancer agents also have proven to be less than adequate for clinical applications. Many of these agents are inefficient or toxic, have significant side effects, result in development of drug resistance or immunosensitization, and are debilitating for the recipient. Moreover, many of these agents depend on ligand surface receptors or on p53/p21 for their effectiveness.

WO 99/07383 which was filed on August 05, 1998 claiming among other a priority date of August 05, 1997 and which was published on February 18, 1999 describes a composition comprising *Mycobacterium phlei-DNA,* wherein this DNA inhibits proliferation of and induces apoptosis in responsive cells and stimulates responsive cells of the immune system to produce bioactive molecules.

Therefore, there is a need for a novel therapeutic agent that inhibits proliferation of and induces apoptosis in bladder cancer cells. This therapeutic agent should be useful as an anti-bladder cancer cell agent and as an adjunct to other anti-bladder cancer cell agents. By adjunct is meant useful with other anti-bladder cancer cell agents to increase treatment effectiveness. Further, such a therapeutic agent should be simple and relatively inexpensive to prepare, its activity should be reproducible among preparations, its activity should remain stable over time, and its effects on bladder cancer cells should be achievable with dose regimens that are associated with minimal toxicity.

### SUMMARY OF THE INVENTION

The present invention satisfies the above needs by the use of deproteinized delipidated Mycobacterium phlei cell wall (MCC) for the manufacture of a medicament for treatment of urinary bladder cancer in an animal including human having urinary bladder cancer.

In this respect, it is noted that MC Pillion et al., British Journal of Cancer (1999) 79 (2), pages 229-235 is a reference that is published after the priority date of the present application and which describes that *Mycobacterium phlei* cell wall complex directly induces apoptosis in human bladder cancer cells.

More particularly, the present invention satisfies the above needs by the use for the manufacture of a medicament of a *Mycobacterium phlei* (*M. phlei*)-DNA (M-DNA)-*M*. *phlei* cell wall complex (MCC), wherein the M-DNA is preserved and complexed on the *M. phlei* cell wall, such that the MCC is effective in inhibiting proliferation of and in inducing apoptosis in bladder cancer cells. MCC is simple and relatively inexpensive to prepare, its activity is reproducible among preparations, it remains therapeutically stable over time, and it is effective at dose regimens that are associated with minimal toxicity even upon repeated administration.

To prepare MCC, *M. phlei* are grown in liquid medium and harvested. The *M. phlei* are disrupted, and the solid components of the disrupted *M. phlei* are collected by centrifugal sedimentation. The solid components are deproteinized, delipidated, and washed. DNase-free reagents are used to minimize M-DNA degradation during preparation.

MCC, in combination with a pharmaceutically acceptable carrier, is to be administered to an animal, including a human, in a dosage sufficient to prevent, treat and eliminate cancer cells within the bladder. The unexpected and surprising ability of MCC to inhibit proliferation of and induce apoptosis in bladder cancer cells including, but not limited to, p53/21 abnormal and drug resistant cells addresses a long felt unfulfilled need in the medical arts and provides an important benefit.

MCC also is effective as an adjunct to enhance the effectiveness of other anticancer agents. These include, but are not limited to, drugs; immunostimulants; antigens; antibodies; vaccines; radiation; chemotherapeutic agents; nucleic acid agents; biologically engineered agents; chemically synthesized agents; agents that target cell death molecules for activation or inactivation; and, agents that inhibit proliferation of and induce apoptosis in responsive cells.

Accordingly it is an object of the present invention to provide a medicament effective to prevent bladder cancer.

Another object of the present invention is to provide a composition medicament effective to treat bladder cancer.

Another object of the present invention is to provide a medicament effective to eliminate bladder cancer.

Another object of the present invention is to provide a medicament that inhibits proliferation of bladder cancer cells.

Another object of the present invention to provide a medicament that induces apoptosis in bladder cancer cells.

Another object of the present invention to provide a medicament that stimulates responsive cells of the immune system within the bladder to produce bioactive molecules.

Another object of the present invention is to provide a composition and medicament that is effective in inhibiting angiogenesis in bladder cancer.

Another object of the present invention is to provide a medicament that is effective as an adjunct to other anti-bladder cancer therapies.

Another object of the present invention is to provide a medicament of particle size and formulation that is optimal for recognition by responsive cells

Another object of the present invention is to provide a medicament of particle size and formulation that is optimal for uptake by responsive cells.

Another object of the present invention is to provide a medicament that can be prepared in large amounts.

Another object of the present invention is to provide a medicament that is relatively inexpensive to prepare.

Another object of the present invention is to provide a medicament that has reproducible activity among preparations.

Another object of the present invention is to provide a medicament that remains stable over time.

Another object of the present invention is to provide a medicament that maintains its effectiveness over time.

Another object of the present invention is to provide a medicament that is minimally toxic to the recipient.

These and other objects, features and advantages of the present invention will become apparent after a review of the following detailed description of the disclosed embodiment and the appended claims.

### BRIEF DESCRIPTION OF THE FIGURES

FIG. 1. Inhibition of proliferation of HT-1376, HT-1197, B-16 F1, THP-1, RAW 264.7, Jurkat, HL-60 and HL-60 MX-1 cancer cells by MCC. Results are the mean ± SD of 3 independent experiments
FIG. 2. Inhibition of proliferation of HT-1376, HT-1197, B-16F1, THP-1, RAW 26A.7, Jurkat, HL-60, and HL-60MX-1 bladder cancer cells by *M. phlei*-DNA (2A), MCC-DNA (2B), calf thymus-DNA (2A & 2B) and herring sperm-DNA (2A & 2B). Results are the mean ± SD of 3 independent experiments.
FIG. 3. Inhibition of proliferation of HT-1197 (3A) and HT-1376 (3B) human bladder cancer cells by MCC and LPS. Results are the mean ± SD of 3 independent experiments.
FIG. 4. Induction of DNA fragmentation in HT-1197 (4A) and HT-1376 (4B) human bladder cancer cells by untreated and DNase I treated MCC and by hIL-12. Results shown are for 1 of 3 experiments, each of which gave similar results
FIG. 5. Release of NuMA from HT-1197 and HT-1376 human bladder cancer cells with increasing concentrations of MCC. Results are the mean ± SD of 3 independent experiments.
FIG. 6. Release of NuMA from HT-1197 (6A) and HT-1376 (6B) human bladder cancer cells with 1 µg/ml MCC or with 100 µg/ml MCC over 48 h. Results are the mean ± SD of 3 independent experiments.
Fig. 7 Release of NuMA from HT-1376 human bladder cancer cells with 1 µg/ml untreated and DNase I treated MCC-DNA and MCC. Results are the mean ± SD of 3 independent experiments.
FIG. 8. Percentage LDH release by HT-1197 and HT-1376 human bladder cancer cells as an indicator of MCC cytotoxicity. Results are the mean ± SD of 3 independent experiments.
FIG. 9. Stability of MCC during 6 months of storage.

### DETAILED DESCRIPTION OF THE INVENTION

The present invention refers to the use of deproteinized delipidated Mycobacterium phlei (M. phlei) cell wall (MCC) for the manufacture of a medicament such that the medicament is effective to prevent, treat and eliminate cancer cells in the urinary bladder of an animal, including a human.

The *Mycobacterium* species *M. phlei* is used.

Therefore, the present invention comprises a *M. phlei*-deoxyribonucleic acid (M-DNA)-*M. phlei* cell wall complex (MCC), wherein the M-DNA is preserved and complexed on the *M. phlei* cell wall, such that the MCC is effective to prevent, treat and eliminate cancer cells in the urinary bladder. In MCC, the amount of M-DNA is enriched relative to the amount of M-DNA in an intact *M. phlei* cell. Further, the M-DNA is preserved and is complexed on the *M. phlei* cell wall so that it is more accessible to the responding cells than is the M-DNA within an intact *M. phlei* cell. Although not wanting to be bound by the following hypothesis, it is believed that the M-DNA, in the form of short oligonucleotides, and its physical association with the *M. phlei* cell wall both contribute to optimal expression of MCCs therapeutic activity.

Methods to increase the therapeutic activity of MCC include, but are not limited to, chemically supplementing or biotechnologically amplifying stimulatory sequences or confirmations of DNA derived from the same or different bacterial species and complexing the M-DNA or MCC to natural or synthetic carriers. Further, MCC can be administered before, at the same time as, or after another and-bladder cancer agent to increase therapeutic effectiveness.

Compositions comprising MCC and its pharmaceutically acceptable carrier are prepared by uniformly and intimately bringing into association the MCC with liquid carriers, with solid carriers, or with both. Liquid carriers include, but are not limited to, aqueous carriers, non-aqueous carriers or both. Solid carriers include, but are not limited to, biological carriers, chemical carriers and biotechno logically engineered carriers.

Among its pharmaceutically acceptable carriers, MCC may be administered in aqueous suspension, oil emulsion, water in oil emulsion, water-in-oil-in-water emulsion, liposomes, microparticles, site-specific emulsions, long-residence emulsions, sticky-emulsions, microemulsions, nanoemulsions, microspheres, nanospheres, nanoparticles, minipumps, and with various natural or synthetic polymers that allow for sustained release of MCC. Further, MCC can be used with any one, all, or any combination of excipients regardless of the carrier used to present MCC to the responding cells. These include, but are not limited to, anti-oxidants, buffers, and bacteriostats, and may include suspending agents and thickening agents.

Preferably, MCC is to be administered as an aqueous suspension. MCC is suspended in a pharmaceutically acceptable carrier such as, but not limited to, DNase-free water, saline or phosphate buffered saline (PBS) and is emulsified by sonication. Optionally, the emulsified mixture is homogenized by microfluidization. For example, lyophilized MCC is suspended in DNase-free water and is sonicated at 20% output for 5 minutes (Model W-385 Sonicator, Heat Systems-Ultrasonics Inc). The sonicated composition is homogenized by microfluidization at 15,000-30,000 psi for one flow-through (Model M-110Y; Microfluidics, Newton, MA). The mixture is either asceptically processed or terminally sterilized.

For administration in a non-aqueous carrier, MCC is emulsified with a mineral oil or with a neutral oil such as, but not limited to, a diglyceride, a triglyceride, a phospholipid, a lipid, an oil and mixtures thereof, wherein the oil contains an appropriate mix of polyunsaturated and saturated fatty acids. Examples include, but are not limited to, soybean oil, canola oil, palm oil, olive oil and myglyol, wherein the number of fatty acid carbons is between 12 and 22 and wherein the fatty acids can be saturated or unsaturated. Optionally, charged lipid or phospholipid can be suspended in the neutral oil. For example, DNase free phosphatidylcholine is added to DNase free triglyceride soybean oil at a ratio of 1 gram of phospholipid to 20 ml of triglyceride and is dissolved by gentle heating at 50°-60° C. Several grams of MCC are added to a dry autoclaved container and the phospholipid-triglyceride solution is added at a concentration of 20 ml per 1 gram of MCC. The suspension is incubated for 60 min. at 20° C and is then mixed with DNase-free PBS in the ratio of 20 ml MCC suspension per liter of PBS. The mixture is emulsified by sonication at 20% output for 5 minutes (Model W-385 Sonicator, Heat Systems-Ultrasonics Inc.). Optionally, the emulsified MCC mixture is homogenized by microfluidization at 15.000-30,000 psi for one flow-through (Model M-110Y: Microfluidics). The MCC emulsion is transferred to an autoclaved, capped bottle for storage at 4° C.

The size of the MCC particles should be optimal for recognition and uptake by the responsive cells. Preferably, the mean diameter of the MCC particles is between about 10 and about 10,000 nm, more preferably between about 100 and about 1000 nm and most preferably between about 250 and about 600 nm.

The M-DNA content of MCC preferably is between about 0.001 and about 90 mg/100 mg dry MCC, more preferably between about 0.01 and about 40 mg/100 mg dry MCC, most preferably between about 0.1 and about 30 mg/100 mg dry MCC. Also, it is preferable that the protein content be less than about 2 mg/100 mg dry MCC and that the fatty acid content be less than about 2 mg/100 mg dry MCC. Unexpectedly, we found that at least about 3.6% of the dry weight of MCC is extractable M-DNA.

MCC are to be administered in an amount effective to induce a therapeutic response in responsive cells of the bladder. The dosage of MCC administered will depend on the condition being treated, the particular formulation, and other clinical factors such as weight and condition of the recipient and route of administration. Preferably, the amount of MCC administered is from about 0.00001 to about 100 mg/kg per dose, more preferably from about 0.0001 to about 50 mg/kg per dose, and most preferably from about 0.001 to about 10 mg/kg per dose.

Routes of administration include, but are not limited to, oral, intra-venous, intra-lesional, intra-tumoral and intra-bladder. Preferably, MCC are to be administered by instillation into the urinary bladder by, but not limited to, a urinary tract catheter. Other methods for instilling MCC into the urinary bladder are known to those skilled in the art.

Depending on the route of administration, the volume per dose is preferably about 0.001 to about 100 ml, more preferably about 0.01 to about 70 ml, and most preferably about 0.1 to about 40 ml. MCC can be administered in a single dose treatment or in multiple dose treatments on a schedule and over a period of time appropriate to the disease being treated, the condition of the recipient and the route of administration.

When administered into the urinary bladder, depending on the volume administered, the dose should remain in the bladder preferably from about 1 min to about 8 h, more preferably from about 15 min to about 4 h, and most preferably from about 30 min to about 2h.

The following examples will serve to further illustrate the present invention without, at the same time, however, constituting any limitation thereof. On the contrary, it is to be clearly understood that resort may be had to various other embodiments, modifications, and equivalents thereof which, after reading the description herein, may suggest themselves to those skilled in the art without departing from the scope of the appended claims.

Subsequent examples describing M-DNA, MCC-DNA and BBC are indicated for comparison.

### EXAMPLE 1

### Preparation of MCC from Mycobacterium phlei and purification of M-DNA from MCC and from M. phlei

MCC was prepared from *Mycobacterium phlei* (*M. phlei*) (strain 110), M-DNA was purified from MCC (MCC-DNA) and M-DNA was purified from *M*. *phlei* (*M*. *phlei-DNA*) as described in Application No. PCT/CA98/00744. All reagents were selected to enhance conservation of the DNA. Unless stated otherwise, MCC, MCC-DNA and *M*. *phlei*-DNA were resuspended in DNase-free water or in a pharmaceutically acceptable DNase-free buffer and emulsified by sonication. MCC, MCC-DNA and *M. phlei*-DNA did not contain endotoxins as determined using a Limulus amebocyte lysate QCL-1000 kit (Bip Whittaker, Walkersville, MD).

### EXAMPLE 2

### Preparation of bacterial-DNA-bacterial cell wall complex and of bacterial DNA from other bacterial species.

Bacterial DNA-bacterial cell wall complex (BCC) and bacterial-DNA (B-DNA) were prepared from *M. smegmatis, M. fortuitous, Nocardia rubra, Nocardia asteroides, Cornybacterium parvum, M, kansaasii, M tuberculosis and M. bovis* as in Example I.

### EXAMPLE 3

### DNase treament

MCC-DNA and MCC, each containing 1 µg of M-DNA, and Regressin® (US Patent No. 4,744,984) were digested with 1 international unit (IU) of RNase-free DNase I (Life Technologies) for 1 h at 25° C in 20 mM Tris HCl, pH 8.4, 2 mM MgCl₂ and 50 mM KCl. DNase I was inactivated by the addition of EDTA to a final concentration of 2.5 mM and heating for 10 min at 65° C.

### EXAMPLE 4

### Cells and reagents

HT-1197 and HT-1376 human bladder cancer cells were obtained from the American Type Culture Collection (ATCC, Rockville, MD) and were cultured in MEM supplemented with non-essential amino acids and vitamins and containing 10% FCS (MEM-FCS) (Gibco Life Science). HT-1197 are anaplastic transitional bladder carcinoma grade 4 cells developed from a human male. HT-1197 cells are sensitive to chemotherapeutic agents such as, but not limited to, doxorubicin. HT-1376 are anaplastic transitional bladder carcinoma grade 3 cells developed from a human female. HT-1376 cells are p53/p21 abnormal and are resistant to chemotherapeutic agents such as. but not limited to, cisplatin and mitomycin. B-16F1, THP-1, RAW 264.7, Jurkat, #L-60 and HL-60MX-1 cells were obtained from the ATCC and were cultured in the media recommended by the ATCC. Unless stated otherwise, cells were seeded in 6 well flat-bottom tissue culture plates at concentrations between 3 X 10⁵ and 10⁶ cells/ml and were maintained at 37° C in a 5% CO₂ atmosphere.

Calf thymus-DNA, herring sperm-DNA and Escherichia coli lipopolysaccharide (LPS) were obtained from Sigma Chemical Co. (St. Louis, MO). Recombinant human IL-12 (hIL-12) was obtained from R&D Systems (Minneapolis, MN).

### EXAMPLE 5

### Inhibition of cell proliferation

Cell proliferation was determined using dimethylthiazol-diphenyltetrazolium bromide (MTT) reduction (Mosman et al. Journal of Immunological Methods 65:55-63, 1983).

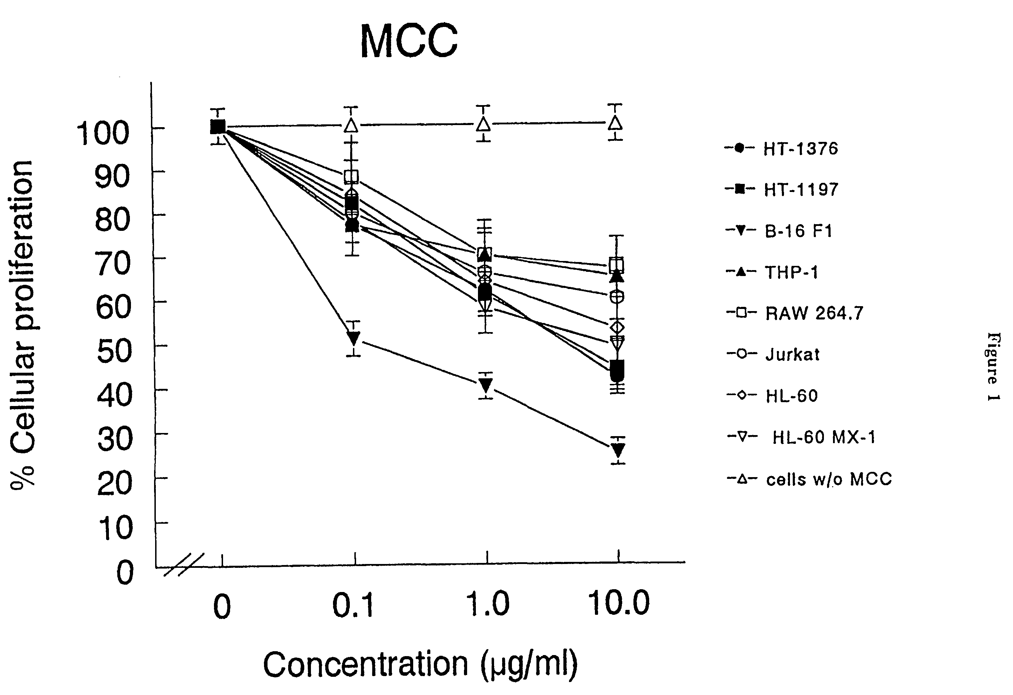

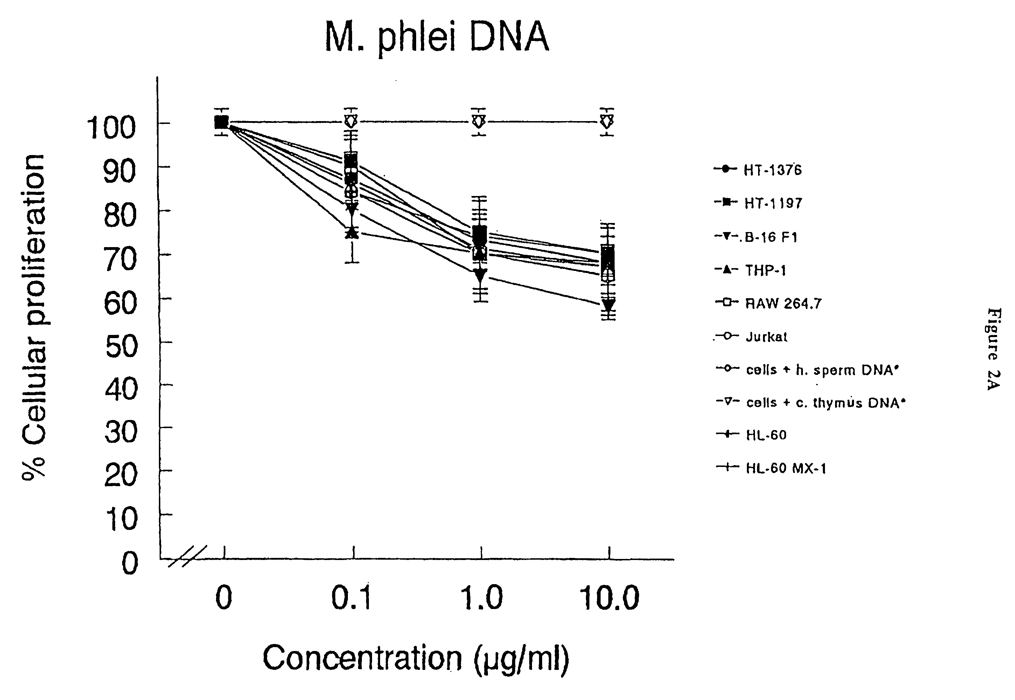

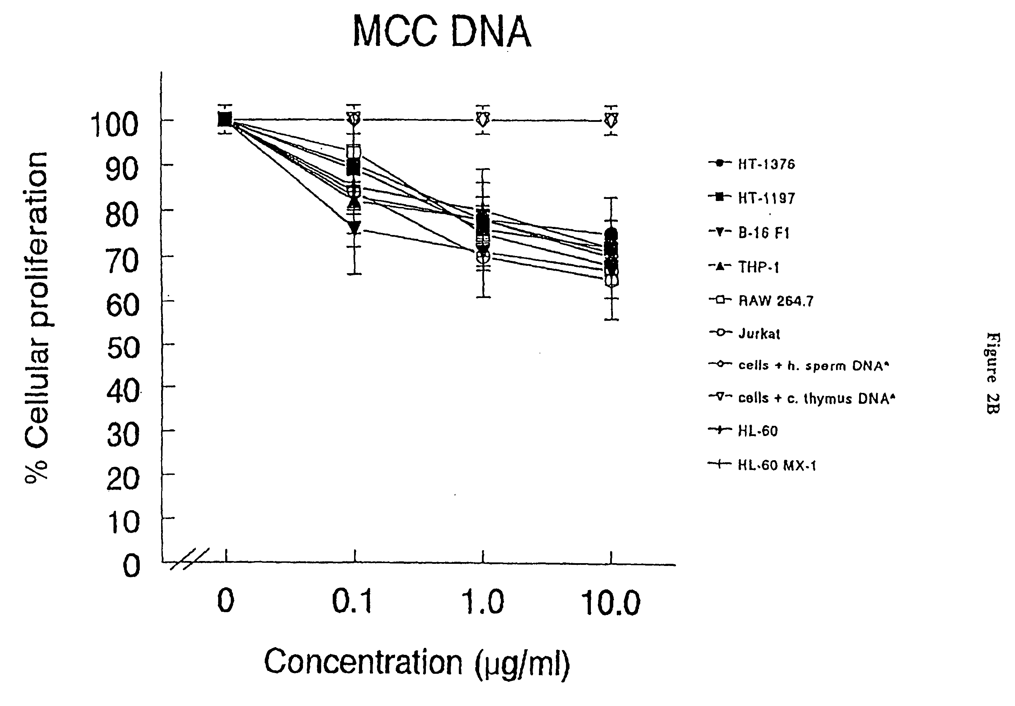

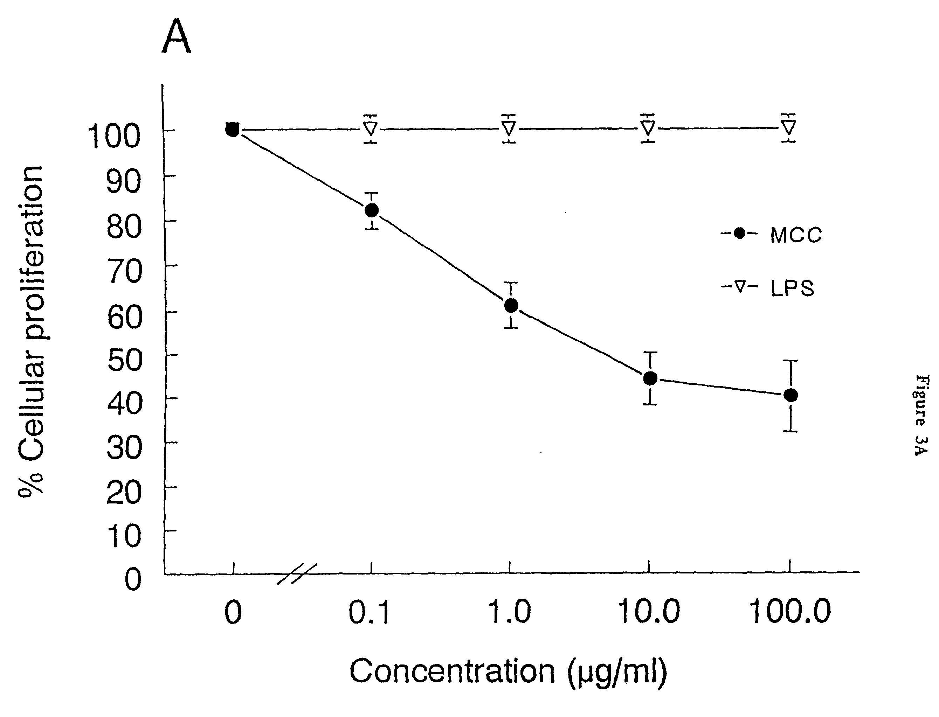

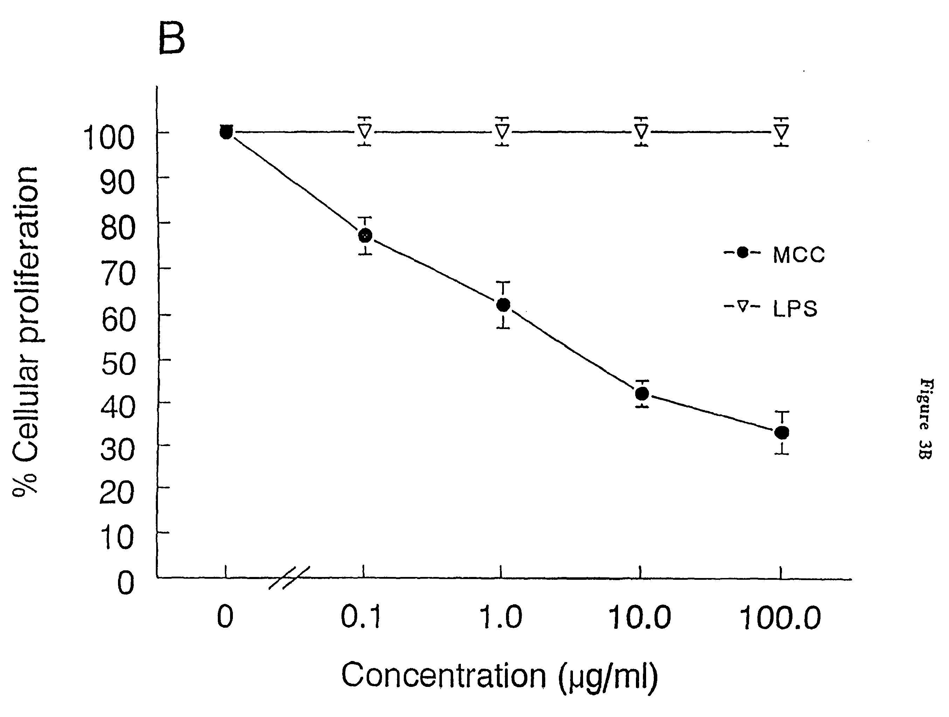

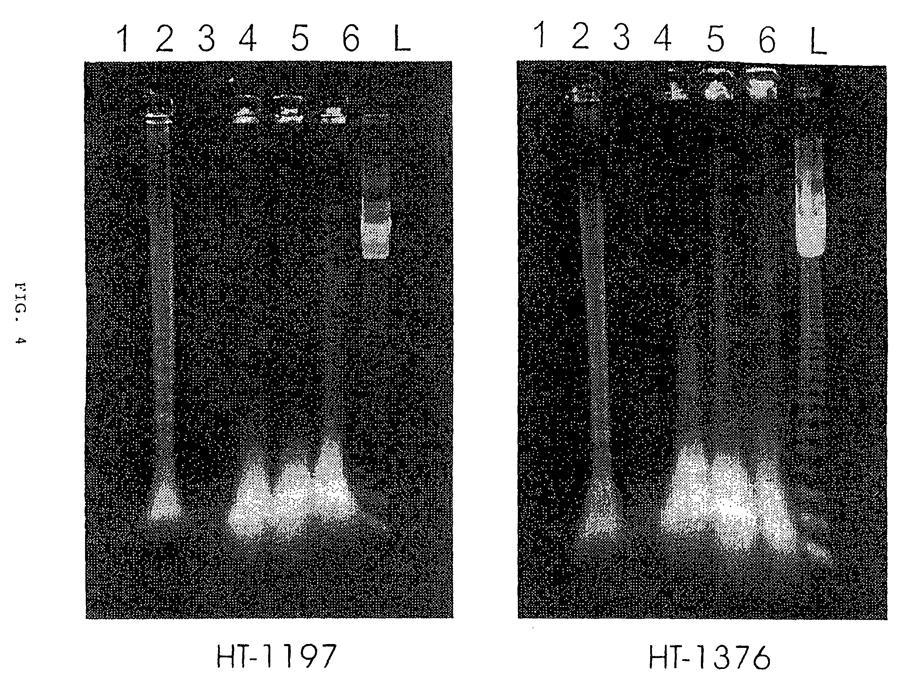

HT-1376, HT-1197, B-16 F1, THP-1, RAW 264.7, Jurkat, HL-60 and HL-60MX-1 cells were incubated for 24 h with from 0 to 10 µg/ml of MCC, *M. phlei*-DNA, MCC-DNA, herring sperm-DNA and calf thymus-DNA. MCC (Fig. 1), *M*. *phlei*-DNA (Fig. 2A) and MCC-DNA (Fig. 2B) inhibited proliferation in a dose dependent manner. Herring sperm-DNA (Figs. 2A & 2B) and calf thymus-DNA (Figs. 2A & 2B) did not inhibit proliferation. HT-1197 (Fig. 3A) and HT-1376 (Fig. 3B) cells also were incubated for 24 h with from 0 to 100 µg/ml of MCC and LPS. MCC inhibited proliferation in a dose dependent manner, whereas LPS did not inhibit proliferation (Figs 3A & 3B).

MCC and M-DNA inhibit proliferation of HT-1197 and of p53/p21 abnormal, drug resistant HT-1376 bladder cancer cells. Further, inhibition of cell proliferation is not common to all DNAs (herring sperm-DNA and calf thymus-DNA) and does not result from nonspecific immunostimulation (LPS).

### EXAMPLE 6

### Induction of apoptosis as indicated by DNA fragmentation

Fragmentation of cellular DNA into nucleosome-sized fragments is characteristic of cells undergoing apoptosis (Newell et al. Nature 357:286-289, 1990). To assess DNA fragmentation, non-adherent cells were collected by centrifugation at 200 g for 10 min. Pellets of non-adherent cells and the remaining adherent cells were lysed with 0.5 ml of hypotonic lysing buffer (10 mM Tris buffer, 1 mM EDTA, 0.2% Triton X-100, pH 7.5). The lysates were centrifuged at 13,000 g for 10 min and the supernatants, containing fragmented DNA, were precipitated overnight at -20° C in 50% isopropanol and 0.5 M NaCl. The precipitates were collected by centrifugation and were analyzed by electrophoresis in 0.7% agarose gels for 3 h at 100V.

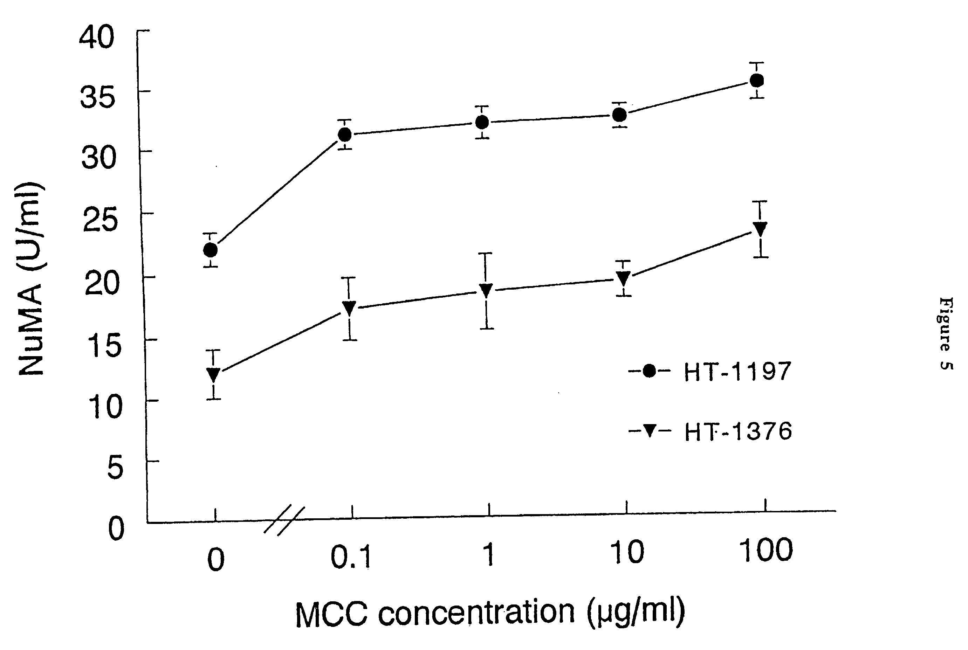

HT-1197 and HT-1376 bladder cancer cells were incubated for 48 h with 1 µg/ml MCC or with 1 ng/ml hIL-12 (Fig. 4). MCC induced significant DNA fragmentation in non-adherent HT-1197 (Fig. 4A, lane 2) and HT-1376 (Fig. 4B. lane 2) cells, but not in adherent HT-1197 (Fig. 4A, lane 3) and HT-1376 (Fig. 4B, lane 3) cells. PBS (Figs. 4A & 4B, lane 5), hIL-12 (Figs. 4A & 4B. lane 4) and DNase 1-treated MCC (Figs. 4A & 4B, lane 7) did not induce DNA fragmentation in non-adherent HT-1197 or HT-1376 cells. Untreated cells (Figs. 4A & 4B, lane 1) showed no DNA fragmentation. A 123-bp DNA ladder (Gibco Life Science) was used to determine the molecular weight of the nucleosome-sized DNA fragments (Figs. 4A & 4B, lane L).

MCC induces apoptosis in HT-1197 and HT-1376 bladder cancer cells, whereas DNase I treated MCC does not induce apoptosis in these cells. This suggests that the intact oligonucleotide structure of the M-DNA is necessary for apoptosis induction, hIL-12 does not induce apoptosis in HT-1197 or in HT-1376 cells.

### EXAMPLE 7

### Induction of apoptosis as indicated by solubilization of nuclear mitotic protein apparatus (NuMA)

Striking morphological changes in the cell nucleus caused by the solubilization and release of NuMA are characteristic of apoptosis. The release of NuMA from cultured cells was determined in units/ml (U/ml) using a commercial ELISA (Calbiochem, Cambridge, MA) (Miller et al. Biotechniques 15:1042-1047, 1993).

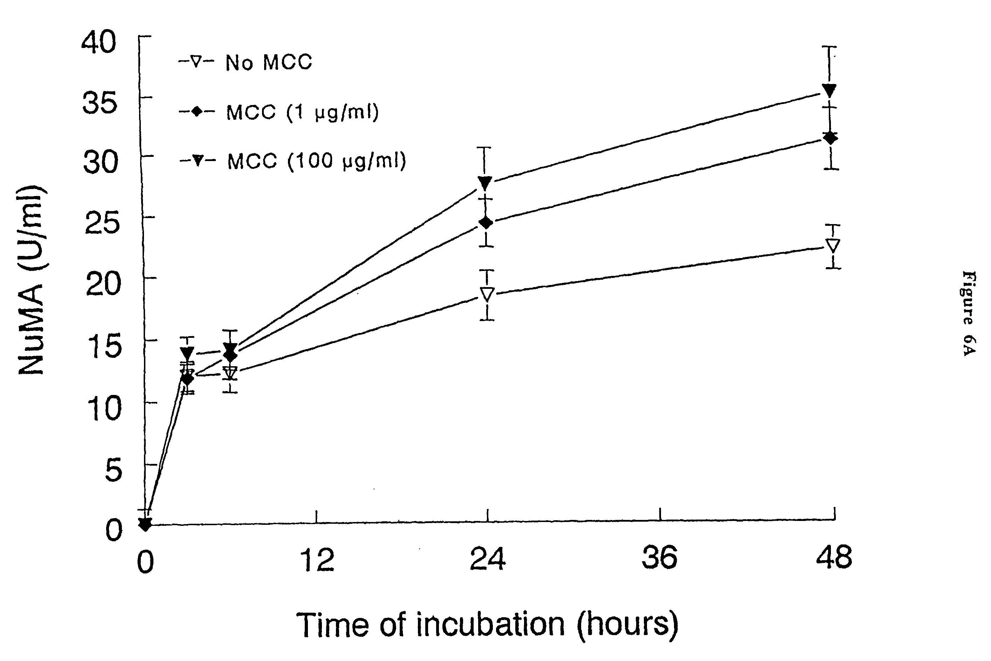

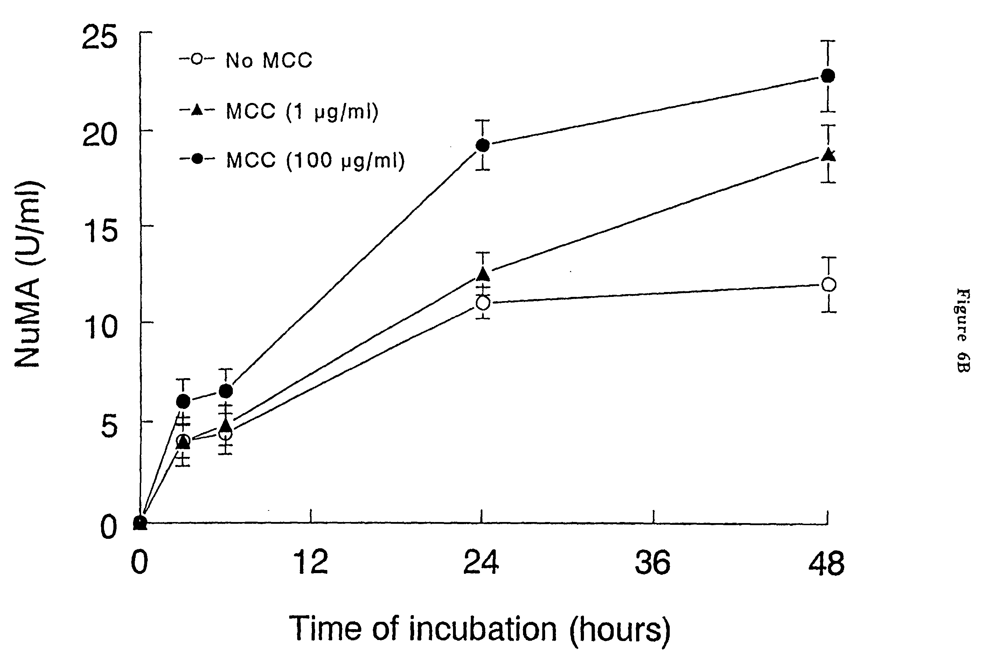

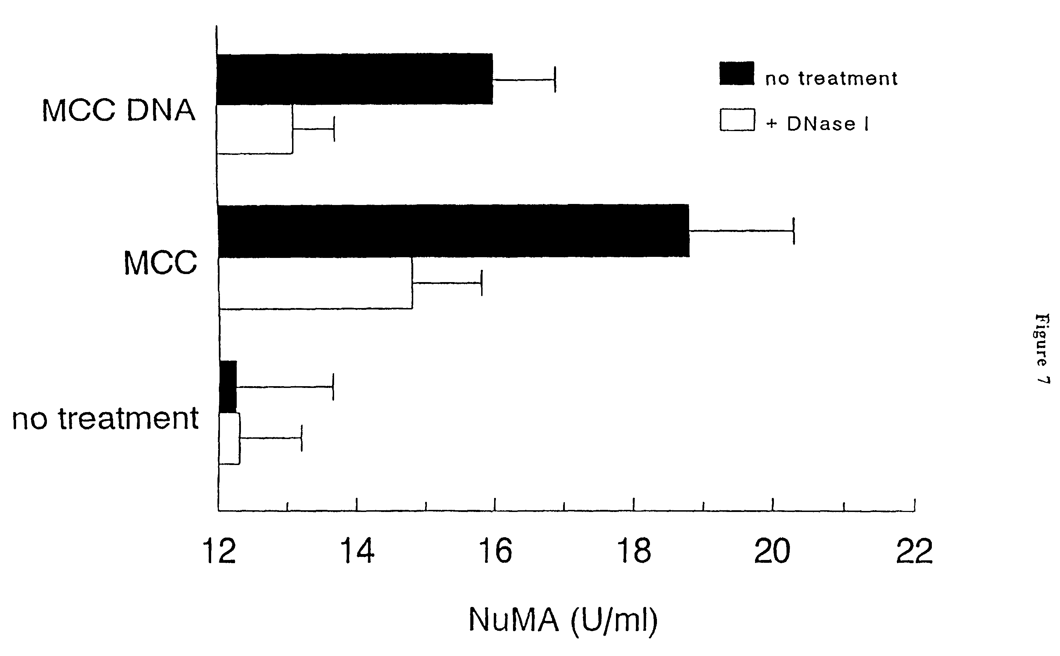

HT-1197 and HT-1376 bladder cancer cells, incubated for 48 h with 0 to 100 µg/ml of MCC, released NuMA in a dose-related manner (Fig. 5). HT-1197 (Fig. 6A) and HT-1376 (Fig. 6B) cells, incubated with 1 µg/ml or with 100 µg/ml of MCC for 48 h, showed enhanced release of NuMA within 24 hours. HT-1197 cells, incubated for 48 h with MCC and with MCC-DNA showed significantly greater NuMA release with MCC than with MCC-DNA (Fig. 7). DNase I treatment of MCC and of MCC-DNA significantly reduced NuMA release (Fig. 7).

MCC induction of apoptosis in HT-1197 and in HT-1376 bladder cancer cells is both time- and dose-dependent. Although not wanting to be bound by the following hypotheses, it is believed that both the oligonucelotide structure of M-DNA and the presentation of M-DNA on *M*. *phlei* cell wall is important for optimal apoptosis induction.

### EXAMPLE 8

### MCC cytotoxicity

Cell cytotoxicity is characterized by the loss of plasma membrane integrity and release of cytoplasmic enzymes such as, but not limited to, LDH (Phillips et al. Vaccine 14:898-904).

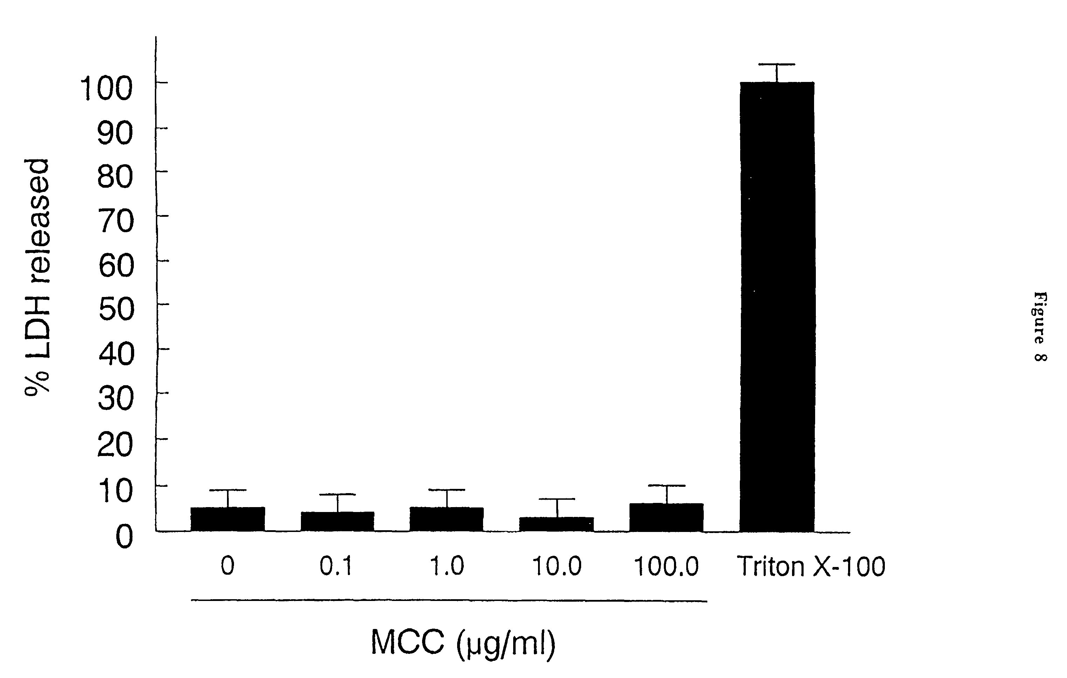

To assess the cytotoxicity of MCC, HT-1197 and HT-1376 human bladder cancer cells were incubated for 48 h with from 0 to 100 µg/ml of MCC or with lysing buffer (10 mM Tris, 1 mM EDTA, 0.2% Triton X-100, pH 7.5) as a control for total LDH release (Filion et al, Biochim Biophys Acta 1329:345-356, 1997). LDH was determined by commercial assay (Sigma-Aldrich). That MCC was not cytotoxic (Fig. 8) demonstrates that MCC acts directly on HT-1197 and HT-1376 bladder cancer cells to inhibit proliferation and to induce apoptosis.

### EXAMPLE 9

### MCC and MCC-DNA treatment of human bladder cancer in nu/nu mice

Human bladder cancer (HT-1197) is established as an ectopic.solid tumor in the subcutaneous tissues of immunodeficient athymic nude mice (nu/nu mice) and the mice are divided into 5 groups. Group 1 receives vehicle alone. Group 2 receives MCC. Group 3 receives DNase I treated MCC. Group 4 receives MCC-DNA. Group 5 receives DNase I treated MCC-DNA. Cancer mass is measured before treatment and weekly during 4 weeks of treatment. Group 2 mice and Group 4 mice show regression of cancer mass. Group 1, 3 and 5 mice do not show regression of cancer mass.

### EXAMPLE 10

### MCC treatment of human bladder cancer

Ten patients with stage 3 orthotopic bladder cancer are divided into 2 groups. Group 1 receives intravesical instillation of MCC weekly for 8 weeks. Group 2 receives standard chemotherapy treatment. Group 1 patients show significant regression of bladder cancer and report no debilitating side effects. Group 2 patients show minimal regression of bladder cancer and report significant debilitating side effects.

### EXAMPLE 11

### MCC stability

MCC at 1 mg/ml was stored as a sterile suspension in 0.85% w/v NaCl in the dark at 4° C or 6 months. Mean particle diameter was calculated using photon correlation spectroscopy (N4 Plus, Coulter Electronics Inc.). The MCC suspension was diluted with 0.85% w/v NaCl to a particle count rate between 5 x10⁴ and 10⁶ counts/sec. Mean particle diameter was calculated in size distribution processor mode (SDP) using the following conditions: fluid refractive index 1.33, temperature 20° C, viscosity 0.93 centipoise, angle of measurement 90.0, sample time 10.5 µs, and sample run time 100 sec. Potential, the electric charge at the hydrodynamic interface between the particles and the bulk solvent, was measured in a Delsa 440SX (Coulter Electronics Inc.) using the following conditions. current 0.7 mA, frequency range 500 Hz, temperature 20° C, fluid refractive index 1.33 viscosity 0.93 centipoise, dielectric constant 78.3, conductivity 16.7 ms/cm, on time 2.5 sec, off time 0.5 sec, and sample run time 60 sec.

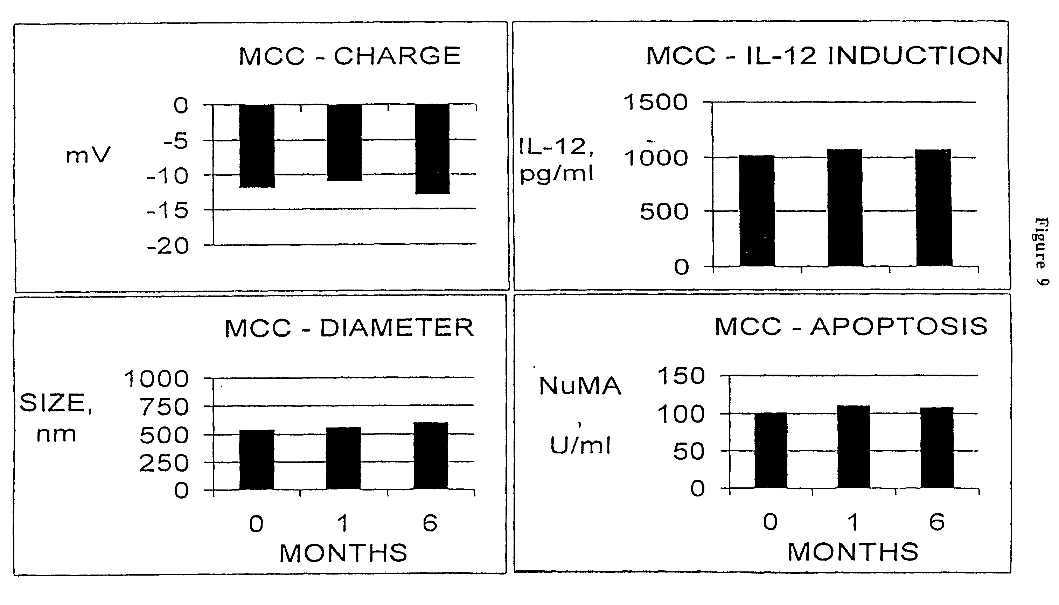

As shown in Fig. 9, MCC charge and MCC diameter remained relatively unchanged during 6 months of storage. Moreover, the MCC stimulation of IL-12 production and induction of apoptosis in THP-1 monocytes remained unchanged during 6 months of storage.

It should be understood, of course, that the foregoing relates only to a preferred embodiment of the present invention and that numerous modifications or alterations may be made therein without departing from the scope of the invention as set forth in the appended claims.

## Claims

1. Use of deproteinized delipidated *Mycobacterium phlei* cell wall (MCC) for the manufacture of a medicament for treatment of urinary bladder cancer in an animal including human having urinary bladder cancer.

2. The use according to claim 1, wherein the MCC induces a response in urinary bladder cancer cells selected from the group consisting of inhibition of proliferation of bladder cancer cells and induction of apoptosis in bladder cancer cells.

3. The use according to claim 2, wherein the urinary bladder cancer cells are drug resistant or p53/21 abnormal.

## Patentansprüche

1. Verwendung von von Proteinen befreiter, von Lipiden befreiter *Mycobakterium phlei* Zellwand (MCC) zur Herstellung eines Arzneimittels zur Behandlung von Harnblasenkrebs in einem Tier, einschließlich eines Menschen, mit Harnblasenkrebs.

2. Verwendung nach Anspruch 1, worin die MCC eine Reaktion in Harnblasenkrebszellen induziert, die aus der Gruppe ausgewählt wird, die aus der Hemmung der Proliferation von Harnblasenkrebszellen und der Induktion der Apoptose in Harnblasenkrebszellen besteht.

3. Verwendung nach Anspruch 2, worin die Harnblasenkrebszellen Arzneimittel-resistent oder p53/21 abnorm sind.

## Revendications

1. Utilisation de paroi cellulaire de *Mycobacterium phlei* (MCC) déprotéinisée et délipidée pour la fabrication d'un médicament destiné au traitement du cancer de la vessie chez un animal, y compris un homme, ayant un cancer de la vessie.

2. Utilisation selon la revendication 1, dans laquelle la MCC induit une réponse dans des cellules du cancer de la vessie sélectionnée dans le groupe constitué d'une inhibition de la prolifération de cellules du cancer de la vessie et d'une induction de l'apoptose de cellules du cancer de la vessie.

3. Utilisation selon la revendication 2, dans laquelle les cellules du cancer de la vessie sont résistantes à des médicaments ou sont anormales pour p53/21.
